(19) Europäisches Patentamt
European Patent Office
Office européen des brevets

(11) Numéro de publication : **0 662 317 A1**

(12) **DEMANDE DE BREVET EUROPEEN**

(21) Numéro de dépôt : **94402535.2**

(51) Int. Cl.$^6$ : **A61K 7/13**

(22) Date de dépôt : **09.11.94**

(30) Priorité : **22.12.93 FR 9315486**

(43) Date de publication de la demande : **12.07.95 Bulletin 95/28**

(84) Etats contractants désignés : **AT BE CH DE ES FR GB IT LI NL SE**

(71) Demandeur : **L'OREAL**
**14, rue Royale**
**F-75008 Paris (FR)**

(72) Inventeur : **Audousset, Marie-Pascale**
**1, Allée Louis Jouvet**
**F-92600 Asnieres (FR)**
Inventeur : **Cotteret, Jean**
**15, allée des Meuniers**
**F-78480 Verneuil S/Seine (FR)**

(74) Mandataire : **Andral, Christophe André Louis**
**L'OREAL**
**Centre de Recherche Charles Zviak**
**Département Propriété Industrielle**
**90, rue du Général Roguet**
**F-92583 Clichy Cedex (FR)**

(54) **Composition de teinture d'oxydation des fibres kératiniques comprenant un 3-fluoroparaaminophénol, un métaaminophénol et/ou une métaphenylènediamine et procédé de teinture utilisant une telle composition.**

(57) L'invention concerne une composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, caractérisée en ce qu'elle contient, à titre de précurseur de colorant d'oxydation, le 3-fluoroparaaminophénol ou l'un de ses sels d'addition avec un acide, et à titre de coupleur, au moins un métaamino-phénol et/ou une métaphénylènediamine spécifique. Elle concerne également l'utilisation de cette composition pour la teinture des fibres kératiniques, notamment les cheveux.

EP 0 662 317 A1

La présente invention concerne une composition de teinture d'oxydation des fibres kératiniques et en particulier des fibres kératiniques humaines, comprenant en association, au moins un 3-fluoroparaaminophénol et au moins un coupleur de type métaaminophénol ou métaphénylènediamine de formule (I) décrite ci-dessous.

Il est connu de teindre les fibres kératiniques et en particulier les cheveux humains, avec des compositions de teinture contenant des précurseurs de colorants d'oxydation, en particulier des ortho- ou paraphénylènediamines, des ortho- ou paraaminophénols, généralement appelés "bases d'oxydation", et des coupleurs encore appelés modificateurs de coloration, plus particulièrement des métaphénylènediamines, des métaaminophénols et des métadiphénols, qui permettent de modifier et d'enrichir en reflets les colorations "de fond" obtenues par les produits de condensation des bases d'oxydation.

De nos jours, on observe un fort engouement pour des colorations à reflets cuivré, acajou ou rouge. Jusqu'ici, ces nuances ont été obtenues avec des teintures à base de paraaminophénol associé à des coupleurs spécifiques. Mais actuellement, l'utilisation du paraaminophénol est remise en question pour des raisons toxicologiques.

La substitution du paraaminophénol est d'autant plus problématique que pour être tout à fait satisfaisantes, les teintures doivent également résister à la lumière, aux lavages, aux intempéries, à la transpiration et aux différents traitements que peuvent subir les cheveux.

Pour remplacer le paraaminophénol, on a déjà proposé d'utiliser le 3-méthylparaaminophénol tel qu'on l'a décrit dans le brevet US-3210252. Ce produit est néanmoins insuffisamment soluble dans les milieux de teinture, engendre une recristallisation dans les compositions, et ne permet finalement pas d'obtenir les nuances cuivrées recherchées.

Il est connu, par ailleurs, selon le brevet français FR-1472078, de réaliser des teintures d'oxydation avec le 3-fluoroparaaminophénol, mais les nuances obtenues sont jaunes et ne conduisent pas aux nuances à reflets cuivré, acajou ou rouge.

Or, la demanderesse, après d'importantes recherches, vient maintenant de découvrir, qu'en associant le 3-fluoroparaaminophénol à des coupleurs de formule (I) donnée dans la suite du texte, il est possible d'obtenir des nuances à reflets cuivré, acajou ou rouge, intenses, satisfaisant aux critères de résistance évoqués antérieurement et ceci tout en évitant les problèmes rencontrés avec le paraaminophénol et son dérivé 3-méthylé, notamment les problèmes de toxicologie et de recristallisation.

La présente invention a ainsi pour objet une composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, caractérisée en ce qu'elle contient à titre de précurseur de colorant d'oxydation, le 3-fluoroparaaminophénol ou l'un de ses sels d'addition avec un acide, et à titre de coupleur, au moins un composé de formule (I) suivante :

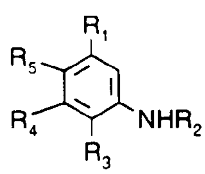

(I)

dans laquelle $R_1$ désigne un radical amino ou hydroxyle, étant entendu que :

- lorsque $R_1$ désigne un radical amino, $R_2$ représente un atome d'hydrogène, un radical alkyle, mono- ou polyhydroxyalkyle, $R_3$ représente un atome d'hydrogène, un radical alkyle ou monohydroxyalcoxy, $R_4$ représente un atome d'hydrogène ou un radical alkyle, $R_5$ représente un radical alcoxy, aminoalcoxy, mono- ou polyhydroxyalcoxy, un radical 2,4-diaminophénoxyalcoxy,
- lorsque $R_1$ désigne un radical hydroxyle, $R_2$ représente un atome d'hydrogène, un radical alkyle, mono- ou polyhydroxyalkyle, $R_3$ représente un atome d'hydrogène, un radical alkyle, alcoxy ou un atome d'halogène, $R_4$ représente un atome d'hydrogène, $R_5$ représente un atome d'hydrogène, un radical alkyle, alcoxy, mono- ou polyhydroxyalkyle, mono- ou poly-hydroxyalcoxy,

les radicaux alkyle ou alcoxy ci-dessus contenant de 1 à 4 atomes de carbone, les radicaux mono- ou polyhydroxyalkyle et mono- ou poly-hydroxyalcoxy ci-dessus désignant des radicaux alkyle ou alcoxy contenant 2 à 3 atomes de carbone et comportant de 1 à 3 groupements hydroxyle, l'atome d'halogène désignant le Chlore, le Fluor ou le Brome,

ou l'un des sels d'addition de ces composés de formule (I) avec un acide.

Les compositions de teinture selon l'invention permettent d'obtenir des nuances à reflets cuivré, acajou ou rouge, intenses, et sont de surcroît des teintures non allergisantes, qui résistent de façon remarquable à la lumière, aux lavages, aux intempéries, et aux différents traitements que peuvent subir les cheveux. Elles sont

tout particulièrement très résistantes à la transpiration.

Un autre objet de l'invention porte sur la composition prête à l'emploi, contenant les différents agents utilisés pour la teinture des fibres kératiniques définis ci-dessus et un agent oxydant, en milieu alcalin ou acide.

L'invention vise également un procédé de teinture des fibres kératiniques , et en particulier des fibres kératiniques humaines telles que les cheveux, consistant à appliquer sur ces fibres au moins une composition (A) contenant, dans un milieu approprié pour la teinture, au moins un précurseur de colorant d'oxydation et au moins un coupleur tels qu'ils ont été définis ci-avant, la couleur étant révélée à pH alcalin, neutre ou acide à l'aide d'un agent oxydant ajouté juste au moment de l'emploi à la composition (A) ou présent dans une composition (B) appliquée simultanément ou séquentiellement de façon séparée.

L'invention a également pour objet des dispositifs de teinture ou "kits" à plusieurs compartiments dont le premier contient au moins le 3-fluoroparaaminophénol à titre de précurseur de colorant d'oxydation et au moins un coupleur de formule (I), et le deuxième, un agent oxydant.

D'autres objets de l'invention apparaîtront à la lecture de la description et des exemples qui suivent.

Les sels d'acide utilisés selon l'invention sont choisis de préférence parmi les chlorhydrates, les sulfates, les bromhydrates et les tartrates.

Le 3-fluoroparaaminophénol ainsi que ses sels sont présents dans des proportions comprises entre 0,005 et 3 % en poids environ par rapport au poids total de la composition de teinture, et de préférence entre 0,05 et 2 % environ.

Parmi les coupleurs de formule (I), on préfère des métaaminophénols choisis parmi le 5-amino 1-hydroxy 2-méthoxy benzène, le 5-amino 1-hydroxy 2-(β-hydroxyéthyloxy) benzène, le 1-hydroxy 5-(β-hydroxyéthylamino) 4-méthoxy 2-méthyl benzène, le 5-amino 1-hydroxy 4-méthoxy 2-méthyl benzène, le 5-amino 4-chloro 1-hydroxy 2-méthyl benzène, le 5-amino 2,4-diméthoxy phénol, le 1-hydroxy 5-(γ-hydrnxyprnpylamino) 2-méthyl benzène,

et des métaphénylènediamines choisies parmi le 3,5-diamino 1-éthyl 2-méthoxy benzène, le 3,5-diamino 2-méthoxy 1-méthyl benzène, le 2,4-diamino 1-éthoxy benzène, le 1,3-bis-(2,4-diaminophénoxy) propane, le 1,3-bis-(2,4-diaminophénoxy) méthane, le 1-(β-aminoéthyloxy) 2,4-diamino benzène, le 2-amino 1-(β-hydroxyéthyloxy) 4-méthylamino benzène, le 1,3-diamino 4,6-bis-(β-hydroxyéthyloxy) benzène, le 2,4-diamino 1-éthoxy 5-méthyl benzène, le 2,4-diamino 5-(β-hydroxyéthyloxy) 1-méthyl benzène, le 2,4-diamino 1-(β,γ-dihydroxypropyloxy) benzène.

Plus particulièrement encore, on préfère des composés de formule (I) dans laquelle $R_3$ et $R_4$ représentent un atome d'hydrogène et notamment des métaaminophénols tels que le 5-N-(β-hydroxyéthylamino) 2-méthyl phénol et le 5-amino 2-méthyl phénol et des métaphénylènediamines telles que le 2,4-diamino 1-(β-hydroxyéthyloxy) benzène et le 2-amino 4-(β-hydroxyéthylamino) 1-méthoxy benzène.

Ces coupleurs de formule (I) ainsi que leurs sels sont présents dans des proportions comprises entre 0,001 et 3 % en poids environ par rapport au poids total de la composition de teinture et de préférence entre 0,05 et 2 % environ.

L'ensemble 3-fluoroparaaminophénol et coupleur(s) de formule (I) représente de 0,006 à 5% en poids environ, et de préférence de 0,1 à 3% en poids environ, par rapport au poids total de la composition.

L'agent oxydant est choisi de préférence parmi le peroxyde d'hydrogène, le peroxyde d'urée, les bromates de métaux alcalins, les persels tels que les perborates et les persulfates. Le peroxyde d'hydrogène est particulièrement préféré.

La composition (A), qui renferme l'association des colorants telle que décrite ci-dessus peut avoir un pH compris entre 3 et 10,5 qui peut être ajusté à la valeur choisie au moyen, soit d'agents alcalinisants habituellement utilisés en teinture des fibres kératiniques, tels que l'ammoniaque, les carbonates alcalins, les alcanolamines telles que par exemple les mono-, di - et triéthanolamines ainsi que leurs dérivés, les hydroxydes de sodium ou de potassium, les composés de formule (II) :

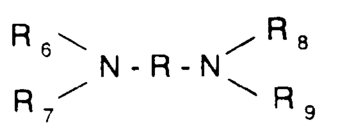

(I)

dans laquelle R est un reste propylène éventuellement substitué par un groupement hydroxyle ou un radical alkyle en $C_1$-$C_4$ ; $R_6$, $R_7$, $R_8$ et $R_9$ simultanément ou indépendamment l'un de l'autre, représentent un atome d'hydrogène, un radical alkyle en $C_1$-$C_4$ ou hydroxyalkyle en $C_1$-$C_4$, soit au moyen d'agents acidifiants classiques, tels que les acides minéraux ou organiques comme par exemple les acides chlorhydrique, tartrique, citrique et phosphorique.

Le pH de la composition (B) renfermant l'agent oxydant tel que défini ci-dessus, est tel qu'après mélange avec la composition (A), le pH de la composition appliquée sur les fibres kératiniques humaines varie de préférence entre 3 et 11. Il est ajusté à la valeur désirée à l'aide d'agents acidifiants ou éventuellement alcalinisants bien connus de l'état de la technique, tels que décrits ci-dessus.

La composition oxydante (B) est de préférence constituée par une solution d'eau oxygénée.

Selon un mode de réalisation préféré du procédé de teinture de l'invention, on mélange au moment de l'emploi, la composition de teinture (A) décrite ci-dessus avec une solution oxydante en une quantité suffisante pour développer une coloration. Le mélange obtenu est ensuite appliqué sur les fibres kératiniques humaines et on laisse poser pendant 5 à 40 minutes, de préférence 15 à 30 minutes, après quoi on rince, on lave au shampooing, on rince à nouveau et on sèche.

Les compositions de teinture peuvent encore contenir, en plus des colorants définis ci-dessus, d'autres coupleurs et/ou des colorants directs, notamment pour modifier les nuances ou les enrichir en reflets, ainsi que des bases d'oxydation autres que le 3-fluoroparaaminophénol.

Ces coupleurs sont bien connus en eux-mêmes et sont choisis parmi les métadiphénols, les dérivés hydroxylés du naphtalène, les dérivés du sésamol, les dérivés aminés ou hydroxylés de la benzomorpholine, la 2,6-diaminopyridine ou ses dérivés, les hydroxyindoles dont notamment le 6-hydroxyindole et les aminoindoles.

Les autres bases d'oxydation peuvent être choisies parmi les paraphénylènediamines parmi lesquelles on peut citer notamment, la paraphénylènediamine, la paratoluylènediamine, la 2,6-diméthylparaphénylènediamine, la 2-hydroxyméthylparaphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-di-(β-hydroxyéthyl) paraphénylènediamine, la 4-amino N-(β-méthoxyéthyl) aniline, les orthoaminophénols et les bases hétérocycliques, telles que la 2,4,5,6-tétraaminopyrimidine et la 2,5-diaminopyridine.

Les colorants directs sont de préférence des colorants azoïques, anthraquinoniques ou des dérivés nitrés de la série benzénique.

Les compositions de teinture contiennent également, dans leur forme de réalisation préférée, des agents tensioactifs anioniques, cationiques, non-ioniques, ou amphotères, bien connus de l'état de la technique, ou leurs mélanges, dans des proportions comprises entre environ 0,5 et 55 % en poids, et de préférence entre 2 et 50 % en poids par rapport au poids total de la composition.

Elles peuvent également contenir des solvants organiques. Parmi eux, on peut citer à titre d'exemple, les alcanols inférieurs en $C_1$-$C_4$, tels que l'éthanol et l'isopropanol, le glycérol, les glycols ou éthers de glycols comme le 2-butoxyéthanol, le propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Ces solvants sont présents de préférence dans des proportions comprises entre environ 1 et 40 % en poids, et en particulier entre 5 et 30 % en poids par rapport au poids total de la composition.

On peut aussi ajouter des agents épaississants choisis par exemple parmi l'alginate de sodium, la gomme arabique, les polymères d'acide acrylique éventuellement réticulés, les dérivés de cellulose, les biopolysaccharides tels que la gomme de xanthane, ou des agents épaississants minéraux tels que la bentonite, présents de préférence dans des proportions comprises entre environ 0,1 et 5 %, et en particulier entre 0,2 et 3 % en poids par rapport au poids total de la composition.

Des agents antioxydants peuvent également être introduits. Ils sont choisis en particulier parmi le sulfite de sodium, l'acide thioglycolique, l'acide thiolactique, le bisulfite de sodium, l'acide déhydroascorbique, l'hydroquinone, la 2-méthyl hydroquinone, la tertio-butyl hydroquinone et l'acide homogentisique et sont présents dans des proportions comprises entre environ 0,05 et 1,5 % en poids par rapport au poids total de la composition.

Les compositions de teinture peuvent également contenir d'autres adjuvants cosmétiquement acceptables, tels que par exemple des agents de pénétration, des agents séquestrants, des parfums, des tampons, des agents dispersants, des agents de traitement, des agents de conditionnement, des agents filmogènes, des agents conservateurs, des agents opacifiants.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. Elle peut être conditionnée sous pression en flacon aérosol en présence d'un agent propulseur et former une mousse.

Des exemples concrets illustrant l'invention vont maintenant être donnés. On commencera par définir les tests utilisés pour évaluer les performances des teintures d'oxydation selon l'invention au niveau de leur résistance à la transpiration, à la lumière et aux shampooings.

Résistance à la transpiration :

On utilise une solution de sueur synthétique de composition suivante : 10 g de NaCl, 1 g d'hydrogénophosphate de potassium, 0,25 g d'histidine, de l'acide lactique jusqu'à pH = 3,2 et de l'eau distillée pour compléter à 100 g. Dans un cristallisoir recouvert d'un verre de montre et renfermant cette solution de sueur, on immerge les mèches de cheveux teints qu'on laisse séjourner de 20 à 50 heures à 37° C. On rince ensuite les mèches et on les sèche.

Résistance à la lumière (Xenotest) :

Les cheveux teints sont fixés sur un support (carton ou plastique). Ces supports sont disposés sur des porte-échantillons qui tournent autour d'une lampe Xénon pendant une durée variant de 20 à 80 heures sous un taux d'humidité variant de 25 à 75% HR (Humidité Relative) et à une température de 25° C.

Résistance aux shampooings (machine Ahiba-Texomet) :

On place des mèches de cheveux teints dans un panier que l'on immerge dans une solution d'un shampooing standard. Le panier est soumis à un mouvement de va-et-vient vertical de fréquence variable ainsi qu'à un mouvement de rotation qui reproduisent l'action d'un frottement manuel, ce qui engendre la formation de mousse.

Après 3 minutes d'épreuve, on retire les mèches que l'on rince puis sèche. Les mèches teintes peuvent être soumises à plusieurs épreuves shampooings consécutives.

**Exemple 1:**

On prépare la composition de teinture suivante :

- 3-Fluoroparaaminophénol 0,635 g
- 5-N-(β-hydroxyéthylamino) 2-méthyl phénol 1,25 g
- Paraphénylènediamine 0,27 g
- Alcool oléique polyglycérolé à 2 moles de glycérol 4,0 g
- Alcool oléique polyglycérolé à 4 moles de glycérol à 78 % de MA 5,0 gMA
- Acide oléique 3,0 g
- Amine oléique à 2 moles d'oxyde d'éthylène, vendue sous la dénomination ETHOMEEN 012 par la Société AKZO 7,0 g
- Laurylamino succinamate de diéthylaminopropyle, sel de sodium à 55% de MA 3,0 gMA
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse à 35% de MA 0,4 gMA
- Acétate d'ammonium 0,8 g
- Antioxydant, séquestrant q.s.
- Parfum, conservateur q.s.
- Ammoniaque à 20% de $NH_3$ 10,0 g
- Eau déminéralisée q.s.p. 100 g

Au moment de l'emploi, on mélange cette composition poids pour poids avec de l'eau oxygénée titrant 20 volumes (6% en poids), de pH 3.

On obtient un mélange de pH 9,8.

On applique ce mélange sur des cheveux gris à 90% de blancs, naturels ou permanentés, pendant 30 minutes. Après rinçage, lavage au shampooing, rinçage et séchage, les cheveux sont teints dans une nuance cuivré-rouge.

Cette nuance, exprimée en valeur Munsell, est la suivante:

- sur cheveux naturels 1,5 Y 2,9 / 3,0
- sur cheveux permanentés: 9,65 R 2,4 / 3,5

Cette teinture présente d'excellentes résistances à la transpiration, à la lumière et aux shampooings répétés.

Ainsi, comparativement à une teinture de nuance équivalente obtenue avec une composition de teinture analogue à celle décrite ci-dessus mais dans laquelle le 3-fluoroparaaminophénol est substitué par le paraaminophénol en une quantité équimoléculaire, soit 0,54 g, on obtient au travers des trois tests de résistance décrits ci-avant les dégradations exprimées en SE (indice Nickerson) suivantes (plus cet indice est faible et plus la résistance est élevée) :

| **Composition de teinture avec** | **Nature des cheveux** | **Résistance (indice de Nickerson)** | | |
|---|---|---|---|---|
| | | à la transpiration | à la lumière (40 heures) | à 8 shampooings |
| 3-fluoroparaaminophénol | Naturels | 2,1 | 3,7 | 6,7 |
| 3-fluoroparaaminophénol | Permanentés | 3,1 | 3,5 | 17,5 |
| Paraaminophénol | Naturels | 9,7 | 5,3 | 8,9 |
| Paraaminophénol | Permanentés | 6,5 | 5,8 | 23,0 |

On constate donc une nette amélioration de la résistance à la transpiration, à la lumière et aux shampooings pour la teinture réalisée au moyen de la composition selon l'invention.

**Exemples 2 à 5** :

On prépare les compositions de teinture, conformes à l'invention, suivantes :

- Colorants (voir tableau I) **x** g
- Alcool oléique polyglycérolé à 2 moles de glycérol 4,0 g
- Alcool oléique polyglycérolé à 4 moles de glycérol (78 % de MA) 5,7 gMA
- Acide oléique 3,0 g
- Amine oléique à 2 moles d'oxyde d'éthylène, vendue sous la dénomination ETHOMEEN 012 par la Société AKZO 7,0 g
- Laurylamino succinamate de diéthylaminopropyle, sel de sodium à 55% de MA 3,0 gMA
- Alcool oléique 5,0 g
- Diéthanolamide d'acide oléique 12,0 g
- Propylèneglycol 3,5 g
- Alcool éthylique 7,0 g
- Dipropylèneglycol 0,5 g
- Monométhyléther de propylèneglycol 9,0 g
- Métabisulfite de sodium en solution aqueuse à 35% de MA 0,46 g MA
- Acétate d'ammonium 0,8 g
- Antioxydant, séquestrant, q.s.
- Parfum, conservateur, q.s.
- Ammoniaque à 20% de $NH_3$ 10,0 g
- Eau déminéralisée q.s.p. 100 g

Au moment de l'emploi, on mélange cette composition poids pour poids avec de l'eau oxygénée titrant 20 volumes (6% en poids), de pH 3.

On obtient un mélange de pH 9,8

On applique ce mélange sur des cheveux gris à 90% de blancs, naturels ou permanentés, pendant 30 minutes. Après rinçage, lavage au shampooing, rinçage et séchage, les cheveux sont teints dans les nuances indiquées dans le tableau II ci-après.

## TABLEAU I

| EXEMPLES<br>Colorants (en g) | 2 | 3 | 4 | 5 |
|---|---|---|---|---|
| 3-Fluoroparaaminophénol | 0,381 | 0,381 | 0,381 | 0,7 |
| 5-amino 2-méthyl phénol | 0,369 | | | |
| 2-amino 4-(β-hydroxyéthylamino) 1-métho benzène | | 0,765 | | |
| 2,4-diamino 1 (β-hydroxyéthyloxy) benzèn dichlorhydrate | | | 0,723 | |
| 5-N- (β-hydroxyéthylamino) 2-méthyl phénol | | | | 0,5 |
| 6-Hydroxyindole | | | | 0,4 |

## TABLEAU II

| EXEMPLES | 2 | 3 | 4 | 5 |
|---|---|---|---|---|
| **NUANCES OBTENUES**<br>- Cheveux gris à 90% blancs naturels | Blond foncé cuivré | | marron acajou | Blond doré cuivré |
| - Cheveux gris à 90% blancs permanentés | Châtain clair cuivré intense | Châtain foncé acajou irisé | marron acajou | Cuivré |

Les compositions des exemples 2 à 5 ci-dessus permettent par ailleurs d'obtenir des teintures dont la résistance à la transpiration, à la lumière et aux shampooings est comparable à celle obtenue pour la composition de l'exemple 1 conforme à l'invention.

**Exemple 6** :

On prépare une composition de teinture suivante :

- Fluoroparaaminophénol 0,6 g
- 2- aminophénol 0,1 g
- 5-amino 2-méthyl phénol 0,15 g
- 5-N-(β-hydroxyéthylamino) 2-méthyl phénol 0,77 g

- Alcool cétylstéarylique (C16/C18 50/50) vendu sous la dénomination Cire de Lanette O par la société HENKEL 18 g
- 2-octyl dodécanol 3 g
- Alcool cétylstéarylique (C16/C18 35/65) oxyéthyléné à 15 moles d'oxyde d'éthylène, vendu sous la dénomination Mergital CS 15 par la société Sinnova-HENKEL 3 g
- Laurylsulfate d'ammonium à 30% MA 3,6 g MA
- Polymère cationique décrit et préparé selon le brevet français FR-2.270.846 constitué de motifs récurrents de formule :

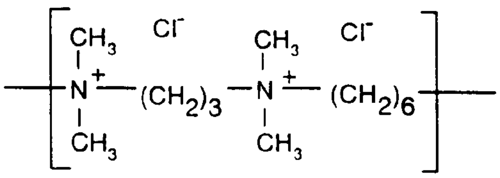

en solution aqueuse à 60% MA 3 g MA
- Ammoniaque à 20% de $NH_3$ 12 g
- Thiolactate d'ammonium à 50% d'acide thiolactique 0,8 g
- Eau déminéralisée q.s.p. 100 g

La composition obtenue est diluée au moment de l'emploi avec 1,5 fois son poids d'eau oxygénée à 20 volumes, dont le pH est 3.

Le mélange ainsi réalisé est appliqué sur des cheveux gris à 90% de blancs, naturels ou permanentés.

Les cheveux sont ensuite rincés, lavés avec un shampooing et séchés.

La coloration obtenue est blond cuivré intense. Elle présente une résistance à la transpiration, à la lumière et aux shampooings comparable à celle obtenue pour la teinture de l'exemple 1 conforme à l'invention.

## Revendications

**1.** Composition de teinture d'oxydation pour fibres kératiniques, en particulier pour fibres kératiniques humaines telles que les cheveux, du type comprenant, dans un milieu approprié pour la teinture, au moins un précurseur de colorant d'oxydation et au moins un coupleur, caractérisée en ce qu'elle contient, à titre de précurseur de colorant d'oxydation, le 3-fluoroparaaminophénol ou l'un de sels d'addition avec un acide, et à titre de coupleur, au moins un composé de formule (I) suivante :

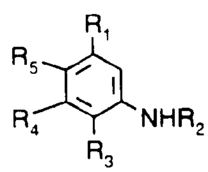

(I)

dans laquelle $R_1$ désigne un radical amino ou hydroxyle, étant entendu que :
- lorsque $R_1$ désigne un radical amino, $R_2$ représente un atome d'hydrogène, un radical alkyle, mono- ou polyhydroxyalkyle, $R_3$ représente un atome d'hydrogène, un radical alkyle ou monohydroxyalcoxy, $R_4$ représente un atome d'hydrogène ou un radical alkyle, $R_5$ représente un radical alcoxy, aminoalcoxy, mono- ou polyhydroxyalcoxy, un radical 2,4-diaminophénoxyalcoxy,
- lorsque $R_1$ désigne un radical hydroxyle, $R_2$ représente un atome d'hydrogène, un radical alkyle, mono- ou polyhydroxyalkyle, $R_3$ représente un atome d'hydrogène, un radical alkyle, alcoxy ou un atome d'halogène, $R_4$ représente un atome d'hydrogène, $R_5$ représente un atome d'hydrogène, un radical alkyle, alcoxy, mono- ou polyhydroxyalkyle, mono- ou poly-hydroxyalcoxy,

les radicaux alkyle ou alcoxy ci-dessus contenant de 1 à 4 atomes de carbone, les radicaux mono- ou polyhydroxyalkyle et mono- ou poly-hydroxyalcoxy ci-dessus désignant des radicaux alkyle ou alcoxy contenant 2 à 3 atomes de carbone et comportant de 1 à 3 groupements hydroxyle, l'atome d'halogène désignant le Chlore, le Fluor ou le Brome,
ou l'un des sels d'addition de ces composés de formule (I) avec un acide.

2. Composition de teinture selon la revendication 1, caractérisée par le fait que le coupleur de formule (I) est un métaaminophénol choisi parmi le 5-N-(β-hydroxyéthylamino) 2 méthyl phénol ou le 5-amino 2-méthyl phénol ou l'un de leurs sels d'addition avec un acide.

3. Composition de teinture selon la revendication 1, caractérisée par le fait que le coupleur de formule (I) est une métaphénylènediamine choisie parmi le 2,4-diamino -1-(β-hydroxyéthyloxy) benzène ou le 2-amino 4-(β-hydroxyéthylamino) 1-méthoxy benzène ou l'un de leurs sels d'addition avec un acide.

4. Composition de teinture selon l'une quelconque des revendications 1 à 3, caractérisée par le fait que les sels d'addition avec un acide sont choisis parmi les chlorhydrates, les sulfates, les bromhydrates et les tartrates.

5. Composition de teinture selon l'une quelconque des revendications 1 à 4, caractérisée par le fait que le 3-fluoroparaaminophénol ou ses sels est présent dans une concentration comprise entre 0,005 et 3 % en poids environ et de préférence entre 0,05 et 2 % en poids environ par rapport au poids total de la composition et que le (s) coupleur (s) de formule (I) ou ses sels, est (sont) présent (s) dans une proportion comprise entre 0,001 et 3 % en poids environ et de préférence entre 0,05 et 2 % en poids environ par rapport au poids total de la composition.

6. Composition de teinture selon l'une quelconque des revendications 1 à 5, prête à l'emploi, caractérisée par le fait qu'elle contient en outre un agent oxydant et qu'elle possède un pH compris entre 3 et 11.

7. Procédé de teinture des fibres kératiniques et en particulier des fibres kératiniques humaines telles que les cheveux, caractérisé par le fait qu'il consiste à appliquer sur les fibres, une composition de teinture (A) selon l'une quelconque des revendications 1 à 6 et à révéler la couleur en milieu acide, neutre ou alcalin à l'aide d'un agent oxydant ajouté juste au moment de l'emploi à cette composition ou présent dans une composition (B) appliquée simultanément ou séquentiellement de façon séparée.

8. Dispositif à plusieurs compartiments ou "kit" de teinture des fibres kératiniques et en particulier des fibres kératiniques humaines telles que les cheveux, caractérisé par le fait qu'il comporte au moins deux compartiments, dont l'un renferme la composition (A) telle que définie dans les revendications 1 à 6 et un autre, la composition (B) comprenant un agent oxydant dans un milieu approprié pour la teinture.

9. Utilisation de la composition de teinture selon les revendications 1 à 6 et du dispositif de teinture ou "kit" à plusieurs compartiments selon la revendication 8 pour la teinture des fibres kératiniques, en particulier des fibres kératiniques humaines telles que les cheveux.

Office européen des brevets

# RAPPORT DE RECHERCHE EUROPEENNE

Numero de la demande
EP 94 40 2535

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,A | FR-A-1 472 078 (HANS SCHWARTZKOPF)<br>* le document en entier *<br>--- | 1,6,7,9 | A61K7/13 |
| A | GB-A-1 048 790 (SCHWARZKOPF VERWALTUNG G M B H)<br>* le document en entier *<br>----- | 1,6,7 | |
| | | | DOMAINES TECHNIQUES RECHERCHES (Int.Cl.6) |
| | | | A61K |

Le présent rapport a été établi pour toutes les revendications

| Lieu de la recherche | Date d'achèvement de la recherche | Examinateur |
|---|---|---|
| LA HAYE | 26 Avril 1995 | Couckuyt, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)